# EUROPEAN PATENT APPLICATION

(11) **EP 4 545 037 A1**
(43) Date of publication of application: **30.04.2025**
(21) Application number: 24207786.5
(22) Date of filing: 21.10.2024
(51) Int. Cl.: A61B 34/37, A61B 90/00, B25J 9/00

(54) **ROBOTIC SURGICAL SYSTEM AND CONTROL METHOD FOR ROBOTIC SURGICAL SYSTEM**

(30) Priority: 24.10.2023 JP 2023182851
(71) Applicant: KAWASAKI JUKOGYO KABUSHIKI KAISHA, Kobe-shi, Hyogo 650-8670 (JP)
(72) Inventor: ICHII, Tetsuo, Kobe-shi, Hyogo, 650-8670 (JP); KOBAYASHI, Ayataka, Kobe-shi, Hyogo, 650-8670 (JP); FUKUNO, Tomohiro, Kobe-shi, Hyogo, 650-8670 (JP); TOJO, Tsuyoshi, Kobe-shi, Hyogo, 650-0047 (JP)
(74) Representative: Hoffmann Eitle

(57) **Abstract**

a robotic surgical system (500) includes a surgical apparatus (100) including a first robot arm (50) to move a medical instrument (1), an arm base (40) to support a proximal end of the first robot arm (50), and a second robot arm (30) to move the arm base (40), and a monitoring controller (360) configured or programmed to monitor whether or not a monitoring point (PO) set with respect to the arm base (40) or the second robot arm (30) is within a monitoring area (AR), and change the monitoring area (AR) based on a posture of the second robot arm (30).

## Description

### BACKGROUND OF THE INVENTION

### Field of the Invention

The present disclosure relates to a robotic surgical system and a control method for a robotic surgical system. Description of the Background Art

Conventionally, a robotic surgical system is known. For example, U.S. Patent Application Publication No. 2014/0249546 discloses a robotic surgical system including a surgical apparatus. The surgical apparatus includes a robot arm that supports a robot instrument, a curved support that supports the robot arm, an arm-shaped member that supports the curved support, and a base unit that supports the arm-shaped member and includes a plurality of wheels. The arm-shaped member has a plurality of degrees of freedom of rotation and can change its posture.

In the robotic surgical system described in U.S. Patent Application Publication No. 2014/0249546, the posture of the arm-shaped member can be changed, and thus depending on the posture of the arm-shaped member, the surgical apparatus may not be able to maintain its balance and may tip. Therefore, it is desired to prevent the surgical apparatus from tipping due to the posture of the arm-shaped member.

### SUMMARY OF THE INVENTION

The present disclosure is intended to provide a robotic surgical system and a control method for a robotic surgical system each capable of preventing a surgical apparatus from tipping due to the posture of a robot arm.

A robotic surgical system according to a first aspect of the present disclosure includes a surgical apparatus including a first robot arm to move a medical instrument, an arm base to support a proximal end of the first robot arm, and a second robot arm to move the arm base, and a monitoring controller configured or programmed to monitor whether or not a monitoring point set with respect to the arm base or the second robot arm is within a monitoring area, and change the monitoring area based on a posture of the second robot arm.

In the robotic surgical system according to the first aspect of the present disclosure, the monitoring controller monitors whether or not the monitoring point set with respect to the arm base or the second robot arm is within the monitoring area. Accordingly, the second robot arm can take a posture within a range in which the surgical apparatus can maintain its balance. Consequently, the surgical apparatus can be prevented from tipping due to the posture of the second robot arm. Furthermore, the monitoring area is changed based on the posture of the second robot arm, and thus the second robot arm can take more postures as compared with a case in which the monitoring area is not changed. Consequently, the second robot arm can take more postures while the surgical apparatus is prevented from tipping due to the posture of the second robot arm.

A control method for a robotic surgical system according to a second aspect of the present disclosure is a control method for a robotic surgical system including a surgical apparatus including a first robot arm to move a medical instrument, an arm base to support a proximal end of the first robot arm, and a second robot arm to move the arm base, and includes monitoring whether or not a monitoring point set with respect to the arm base or the second robot arm is within a monitoring area, and changing the monitoring area based on a posture of the second robot arm.

In the control method for a robotic surgical system according to the second aspect of the present disclosure, as described above, it is monitored whether or not the monitoring point set with respect to the arm base or the second robot arm is within the monitoring area. Accordingly, the second robot arm can take a posture within a range in which the surgical apparatus can maintain its balance. Consequently, it is possible to provide the control method for a robotic surgical system capable of preventing the surgical apparatus from tipping due to the posture of the second robot arm. Furthermore, the monitoring area is changed based on the posture of the second robot arm, and thus the second robot arm can take more postures as compared with a case in which the monitoring area is not changed. Consequently, the second robot arm can take more postures while the surgical apparatus is prevented from tipping due to the posture of the second robot arm.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

FIG. 1 is a diagram showing the configuration of a robotic surgical system according to an embodiment.
FIG. 2 is a diagram showing a display of a medical cart according to the embodiment.
FIG. 3 is a diagram showing the configuration of the medical cart according to the embodiment.
FIG. 4 is a diagram showing the configuration of a positioner according to the embodiment.
FIG. 5 is a diagram showing the configuration of a robot arm according to the embodiment.
FIG. 6 is a diagram showing a pair of forceps.
FIG. 7 is a perspective view showing the configuration of an arm operation unit according to the embodiment.
FIG. 8 is a diagram for illustrating translational movement of the robot arm.
FIG. 9 is a diagram for illustrating rotational movement of the robot arm.
FIG. 10 is a diagram showing an endoscope.
FIG. 11 is a diagram showing a pivot position setting instrument.
FIG. 12 is a diagram showing operation units according to the embodiment.
FIG. 13 is a diagram showing a right-handed wrist according to the embodiment.
FIG. 14 is a diagram showing a left-handed wrist according to the embodiment.
FIG. 15 is a perspective view showing foot pedals according to the embodiment.
FIG. 16 is a control block diagram of the robotic surgical system according to the embodiment.
FIG. 17 is a control block diagram of the robot arm according to the embodiment.
FIG. 18 is a control block diagram of the positioner and the medial cart according to the embodiment.
FIG. 19 is a control block diagram of the operation unit according to the embodiment.
FIG. 20 is a diagram for illustrating a monitoring area according to the embodiment.
FIG. 21 is another diagram for illustrating the monitoring area according to the embodiment.
FIG. 22 is a diagram for illustrating expansion of the monitoring area according to the embodiment.
FIG. 23 is another diagram for illustrating expansion of the monitoring area according to the embodiment.
FIG. 24 is a diagram for illustrating a case in which the monitoring area is not expanded according to the embodiment.
FIG. 25 is a diagram for illustrating the rotation angle of an arm base according to the embodiment.
FIG. 26 is a diagram for illustrating the inclination angle of the arm base according to the embodiment.

### DESCRIPTION OF THE PREFERRED EMBODIMENTS

### Configuration of Robotic Surgical System

The configuration of a robotic surgical system 500 according to this embodiment is now described. The robotic surgical system 500 includes a surgical robot 100, a remote control apparatus 200, a vision unit 300, and an image processing unit 400. The surgical robot 100 and the remote control apparatus 200 are examples of a surgical apparatus and a first operation apparatus, respectively.

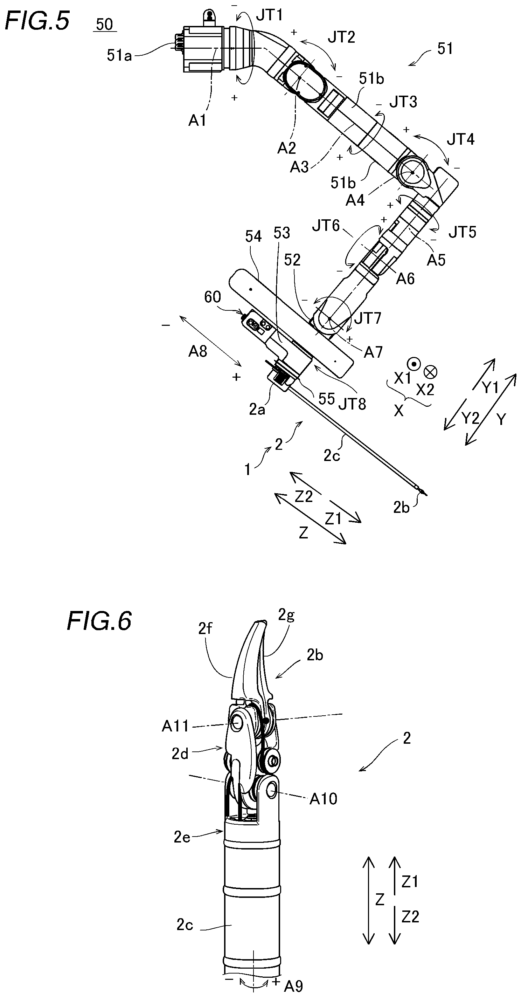

In this specification, as shown in FIGS. 5 to 8, the longitudinal direction of a surgical instrument 1 is defined as a Z direction. The distal end side of the surgical instrument 1 is defined as a Z1 side, and the proximal end side of the surgical instrument 1 is defined as a Z2 side. A direction perpendicular to the Z direction is defined as an X direction. One side in the X direction is defined as an X1 side, and the other side is defined as an X2 side. A direction perpendicular to the Z direction and the X direction is defined as a Y direction. One side in the Y direction is defined as a Y1 side, and the other side is defined as a Y2 side.

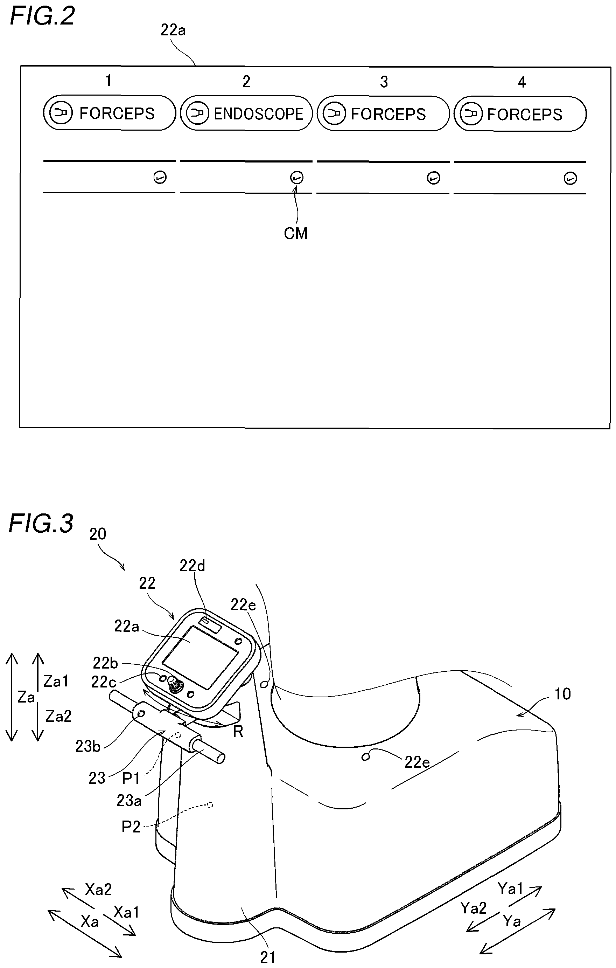

In this specification, as shown in FIG. 3, a right-left direction as viewed by an operator who operates a display 22a of an input 22 is defined as an Xa direction. A right direction is defined as an Xa1 direction, and a left direction is defined as an Xa2 direction. A forward-rearward direction as viewed by the operator who operates the display 22a of the input 22 is defined as a Ya direction. A forward direction is defined as a Ya1 direction, and a rearward direction is defined as a Ya2 direction. A direction perpendicular to a floor surface on which the surgical robot 100 is placed is defined as a Za direction. An upward direction is defined as a Za1 direction, and a downward direction is defined as a Za2 direction.

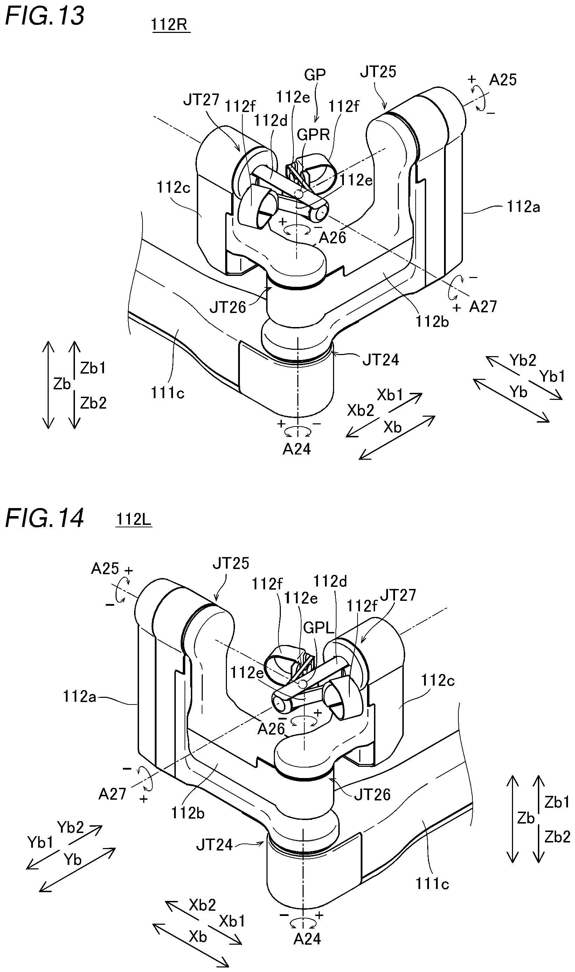

In this specification, as shown in FIGS. 13 and 14, a direction perpendicular to a floor surface on which the remote control apparatus 200 is placed is defined as a Zb direction, the forward-rearward direction of an operator who operates operation units 110, which is perpendicular to the Zb direction, is defined as a Yb direction, and a direction perpendicular to the Zb direction and the Yb direction is defined as an Xb direction. In the Zb direction, an upward direction is defined as a Zb1 direction, and a downward direction is defined as a Zb2 direction. In the Yb direction, one side is defined as a Yb1 direction, and the other side is defined as a Yb2 direction. In the Xb direction, one side is defined as an Xb1 direction, and the other side is defined as an Xb2 direction.

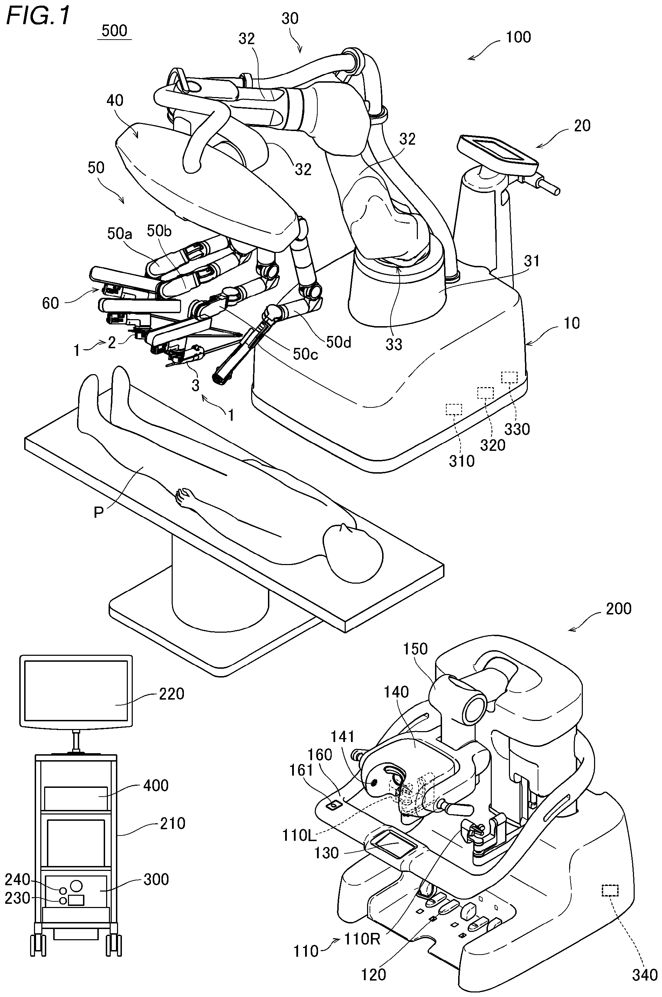

As shown in FIG. 1, the surgical robot 100 is arranged in an operating room. The remote control apparatus 200 is spaced apart from the surgical robot 100. The remote control apparatus 200 receives operations on the surgical instrument 1. Specifically, an operator such as a doctor inputs a command to the remote control apparatus 200 to cause the surgical robot 100 to perform a desired operation. The remote control apparatus 200 transmits the input command to the surgical robot 100.
The surgical robot 100 operates based on the received command. The surgical robot 100 is arranged in the operating room that is a sterilized sterile field. The surgical instrument 1 is an example of a medical instrument.

### Configuration of Surgical Robot

As shown in FIG. 1, the surgical robot 100 includes a medical cart 10, a cart positioner operation unit 20, a positioner 30, an arm base 40, a plurality of robot arms 50, and an arm operation unit 60 provided on each of the robot arms 50. The positioner 30 is an example of a second robot arm. The robot arm 50 is an example of a first robot arm.

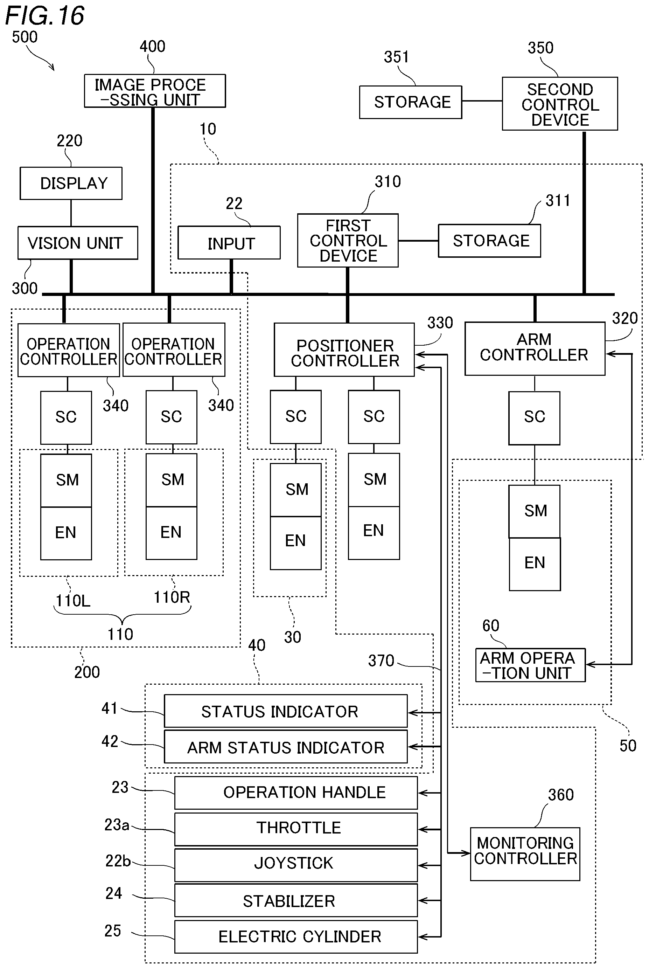

As shown in FIG. 3, the cart positioner operation unit 20 is supported by a cart positioner operation support 21 at the rear of the medical cart 10, and the medical cart 10 or the positioner 30 is moved by operating the cart positioner operation unit 20. The cart positioner operation unit 20 includes the input 22 and an operation handle 23. The input 22 receives operations to move the positioner 30, the arm base 40, and the plurality of robot arms 50 or change their postures mainly in order to prepare for surgery before the surgery. The cart positioner operation unit 20 includes the operation handle 23, and a stabilizer 24 and an electric cylinder 25 shown in FIG. 16. The cart positioner operation unit 20 is an example of a second operation apparatus.

As shown in FIG. 3, the input 22 of the cart positioner operation unit 20 includes the display 22a, a joystick 22b, an enable switch 22c, an error reset button 22d, and speakers 22e. The display 22a is a liquid crystal panel, for example. As shown in FIG. 2, the display 22a displays numbers corresponding to the plurality of robot arms 50. The display 22a also displays the type of surgical instrument 1 attached to each of the plurality of robot arms 50. A check mark CM indicating that a pivot position PP described below has been set is displayed on the display 22a.

As shown in FIG. 3, the joystick 22b is arranged in the vicinity of or adjacent to the display 22a of the input 22 of the cart positioner operation unit 20. The joystick 22b is provided to operate the positioner 30. The positioner 30 is moved three-dimensionally by selecting an operation mode displayed on the display 22a and operating the joystick 22b.

The enable switch 22c is arranged in the vicinity of or adjacent to the joystick 22b of the cart positioner operation unit 20. The enable switch 22c enables or disables movement of the positioner 30. When the joystick 22b is operated while the enable switch 22c is pressed to enable movement of the positioner 30, the positioner 30 is moved.

The error reset button 22d resets errors in the robotic surgical system 500. The errors may be abnormal deviation errors, for example. The speakers 22e are arranged in pair. The pair of speakers 22e are arranged in the vicinity of or adjacent to the location of the positioner 30 in the medical cart 10.

The operation handle 23 is arranged in the vicinity of the display 22a of the cart positioner operation unit 20. The operation handle 23 includes a throttle 23a that is gripped and twisted by an operator such as a nurse or a technician to operate movement of the medical cart 10. Specifically, the operation handle 23 is arranged below the input 22. As the throttle 23a is twisted from the near side to the far side, the medical cart 10 moves forward. As the throttle 23a is twisted from the far side to the near side, the medical cart 10 moves rearward. The speed of the medical cart 10 is changed according to a twisting amount of the throttle 23a. The operation handle 23 is rotatable to the left and right shown by an R direction, and the medical cart 10 is turned with rotation of the operation handle 23.

An enable switch 23b for enabling or disabling movement of the medical cart 10 is provided on the operation handle 23 of the cart positioner operation unit 20. When the throttle 23a of the operation handle 23 is operated while the enable switch 23b is pressed to enable movement of the medical cart 10, the medical cart 10 is moved.

As shown in FIG. 1, the positioner 30 includes a 7-axis articulated robot, for example. The positioner 30 is arranged on the medical cart 10. The positioner 30 adjusts the position and posture of the arm base 40. The positioner 30 moves the arm base 40 three-dimensionally.

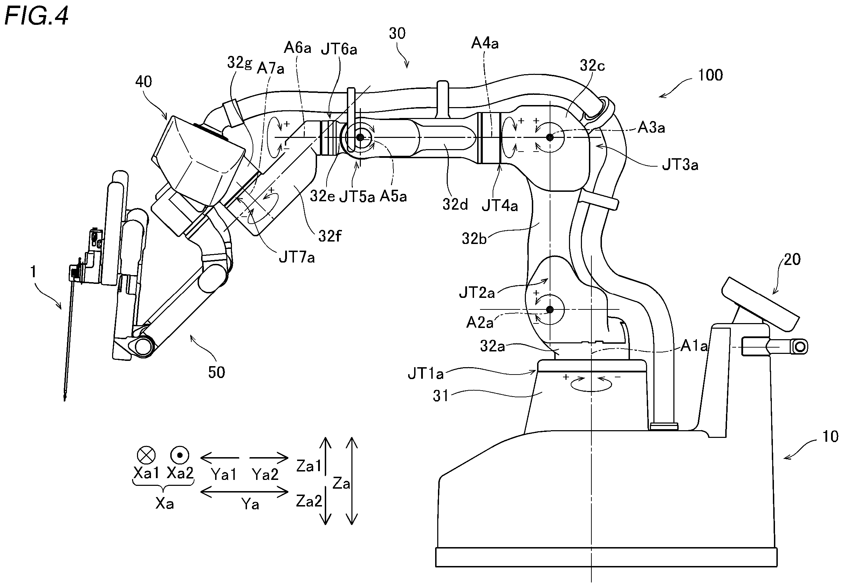

As shown in FIG. 4, the positioner 30 includes a base 31, a plurality of links 32a, 32b, 32c, 32d, 32e, 32f, and 32g, and a plurality of joints JT1a, JT2a, JT3a, JT4a, JT5a, JT6a, and JT7a.

The joints JT1a, JT2a, JT3a, JT4a, JT5a, JT6a, and JT7a have A1a, A2a, A3a, A4a, A5a, A6a, and A7a axes as their respective rotation axes. The joint JT1a rotationally drives the link 32a about the A1a axis extending vertically with respect to the base 31. The joint JT2a rotationally drives the link 32b about the A2a axis in a direction perpendicular to a direction in which the A1a axis extends with respect to the link 32a. The joint JT3a rotationally drives the link 32c about the A3a axis in a direction parallel to a direction in which the A2a axis extends with respect to the link 32b.

The joint JT4a rotationally drives the link 32d about the A4a axis in a direction perpendicular to a direction in which the A3a axis extends with respect to the link 32c. The joint JT5a rotationally drives the link 32e about the A5a axis in a direction perpendicular to a direction in which the A4a axis extends with respect to the link 32d. The joint JT6a rotationally drives the link 32f about the A6a axis in a direction perpendicular to a direction in which the A5a axis extends with respect to the link 32e. The joint JT7a rotationally drives the link 32g about the A7a axis in a direction perpendicular to a direction in which the A6a axis extends with respect to the link 32f.

As shown in FIG. 1, the arm base 40 is attached to a distal end of the positioner 30. Specifically, the arm base 40 is attached to the link 32g of the positioner 30. A proximal end of each of the plurality of robot arms 50 is attached to the arm base 40. Each of the plurality of robot arms 50 is able to take a folded and stored posture. The arm base 40 and the plurality of robot arms 50 are covered with sterile drapes and used. Moreover, each of the robot arms 50 supports the surgical instrument 1.

A status indicator 41 and an arm status indicator 42 that are shown in FIG. 16 are provided on the arm base 40. The status indicator 41 indicates the status of the robotic surgical system 500. The arm status indicator 42 indicates the statuses of the robot arms 50.

The plurality of robot arms 50 are arranged. Specifically, four robot arms 50a, 50b, 50c, and 50d are arranged. The robot arms 50a, 50b, 50c, and 50d have the same or similar configurations as each other.

As shown in FIG. 5, each robot arm 50 includes an arm portion 51, a first link 52, a second link 53, and a translation mechanism 54. The robot arm 50 includes joints JT1, JT2, JT3, JT4, JT5, JT6, JT7, and JT8. The joints JT1, JT2, JT3, JT4, JT5, JT6, and JT7 have A1, A2, A3, A4, A5, A6, and A7 axes as rotation axes, respectively. The joint JT8 has an A8 axis as a linear motion axis. The A1 to A7 axes are rotation axes of the joints JT1 to JT7 of the arm portion 51, respectively. The A7 axis is a rotation axis of the first link 52. The A8 axis is a linear motion axis along which the translation mechanism 54 moves the second link 53 relative to the first link 52 in the Z direction. The arm portion 51 includes a base 51a and links 51b.

The arm portion 51 includes a 7-axis articulated robot arm. The first link 52 is arranged at a distal end of the arm portion 51. The arm operation unit 60 described below is attached to the second link 53. The translation mechanism 54 is arranged between the first link 52 and the second link 53. A holder 55 that holds the surgical instrument 1 is arranged on the second link 53. The translation mechanism 54 translates the holder 55 to which the surgical instrument 1 is attached between a first position and a second position. The first position refers to an end position on the Z2 side in a range of movement along the A8 axis of the holder 55 by the translation mechanism 54. The second position refers to an end position on the Z1 side in the range of movement along the A8 axis of the holder 55 by the translation mechanism 54.

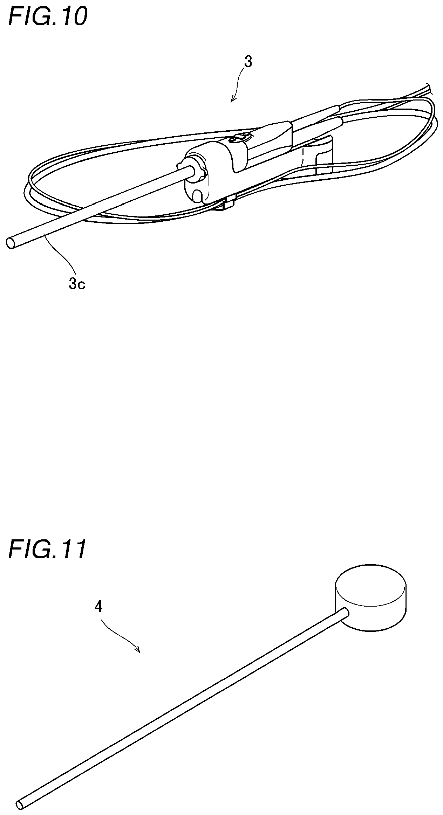

Each of the plurality of robot arms 50 is provided to support the surgical instrument 1. The surgical instrument 1 is attached to a distal end of each of the plurality of robot arms 50. The surgical instrument 1 includes a replaceable instrument 2, an endoscope 3 shown in FIG. 10 to capture an image of a surgical site, and a pivot position setting instrument 4 shown in FIG. 11 to set the pivot position PP, for example. The instrument 2 includes a driven unit 2a, a pair of forceps 2b, and a shaft 2c.

As shown in FIG. 1, the endoscope 3 is attached to the distal end of one of the plurality of robot arms 50, such as the robot arm 50c, and the instrument 2 is attached to the distal end of each of the remaining robot arms 50a, 50b, and 50d, for example. The endoscope 3 is preferably attached to one of two robot arms 50b and 50c arranged in the center among the four robot arms 50 arranged adjacent to each other.

### Configuration of Instrument

As shown in FIG. 6, the pair of forceps 2b is provided at a distal end of the instrument 2, for example. At the distal end of the instrument 2, in addition to the pair of forceps 2b, a pair of scissors, a grasper, a needle holder, a microdissector, a stable applier, a tacker, a suction cleaning tool, a snare wire, a clip applier, etc. are arranged as instruments having joints. At the distal end of the instrument 2, a cutting blade, a cautery probe, a washer, a catheter, a suction orifice, etc. are arranged as instruments having no joint.

The pair of forceps 2b includes a first support 2d and a second support 2e. The first support 2d supports the proximal end sides of jaw members 2f and 2g such that the proximal end sides of the jaw members 2f and 2g are rotatable about an A11 axis. The second support 2e supports the proximal end side of the first support 2d such that the proximal end side of the first support 2d is rotatable about an A10 axis. The shaft 2c rotates about an A9 axis. The jaw members 2f and 2g pivot about the A11 axis to open and close.

### Configuration of Arm Operation Unit

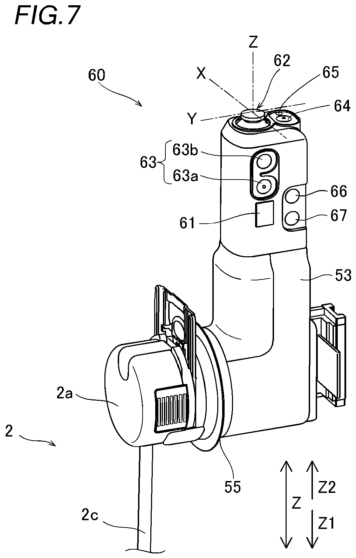

As shown in FIG. 7, the arm operation unit 60 is attached to the robot arm 50 to operate the robot arm 50. Specifically, the arm operation unit 60 is attached to the second link 53.

The arm operation unit 60 includes an enable switch 61, a joystick 62, and linear switches 63, a mode switching button 64, a mode indicator 65, a pivot button 66, and an adjustment button 67.

The enable switch 61 is pressed to enable or disable movement of the robot arm 50 in response to the joystick 62 and the linear switches 63. When the enable switch 61 is pressed by an operator such as a nurse or an assistant grasping the arm operation unit 60, movement of the surgical instrument 1 by the robot arm 50 is enabled.

The joystick 62 is an operation tool to control movement of the surgical instrument 1 by the robot arm 50. The joystick 62 controls a moving direction and a moving speed of the robot arm 50. The robot arm 50 is moved in accordance with a tilting direction and a tilting angle of the joystick 62.

The linear switches 63 are switches to move the surgical instrument 1 in the Z direction, which is the longitudinal direction of the surgical instrument 1. The linear switches 63 include a linear switch 63a to move the surgical instrument 1 in a direction in which the surgical instrument 1 is inserted into a patient P, and a linear switch 63b to move the surgical instrument 1 in a direction in which the surgical instrument 1 is moved away from the patient P. Both the linear switch 63a and the linear switch 63b are push-button switches.

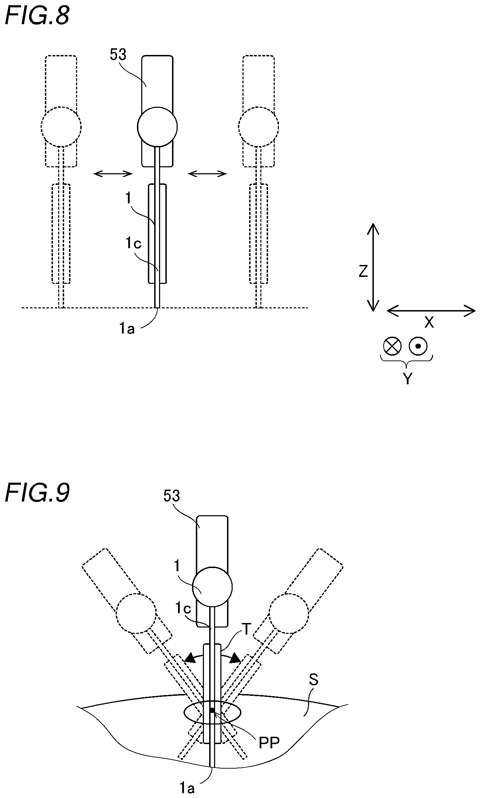

The mode switching button 64 is a push-button switch to switch between a mode for translationally moving the surgical instrument 1 and a mode for rotationally moving the surgical instrument 1. As shown in FIG. 8, in the mode for translationally moving the robot arm 50, the robot arm 50 is moved such that a distal end 1a of the surgical instrument 1 is moved in an X-Y plane. As shown in FIG. 9, in the mode for rotationally moving the robot arm 50, the robot arm 50 is moved such that the surgical instrument 1 is rotationally moved about a center of the A11 axis of the pair of forceps 2b of the instrument 2 as the surgical instrument 1 as a fulcrum when any pivot position PP is not stored in a storage 351, and the surgical instrument 1 is rotationally moved about the pivot position PP as a fulcrum when the pivot position PP is stored in the storage 351. In this case, the surgical instrument 1 is rotationally moved with the shaft 1c of the surgical instrument 1 inserted into a trocar T. The mode switching button 64 is arranged on a Z-direction side surface of the arm operation unit 60.

The mode indicator 65 indicates a switched mode. The mode indicator 65 is on to indicate a rotational movement mode and is off to indicate a translational movement mode. Furthermore, the mode indicator 65 also serves as a pivot position indicator that indicates that the pivot position PP has been set. The mode indicator 65 is arranged on the Z-direction side surface of the arm operation unit 60.

The pivot button 66 is a push-button switch to set the pivot position PP that serves as a fulcrum for movement of the surgical instrument 1 attached to the robot arm 50.

The adjustment button 67 is a button to optimize the position of the robot arm 50. After the pivot position PP for the robot arm 50 to which the endoscope 3 has been attached is set, the positions of the other robot arms 50 and the arm base 40 are optimized when the adjustment button 67 is pressed. The adjustment button 67 is a button different from the enable switch 61.

### Remote Control Apparatus

As shown in FIG. 1, the remote control apparatus 200 is arranged inside or outside the operating room, for example. The remote control apparatus 200 includes the operation units 110, foot pedals 120, a touch panel 130, a monitor 140, a support arm 150, a support bar 160, an error reset button 161. The operation units 110 includes operation handles for the operator such as a doctor to input a command.

### Operation Unit

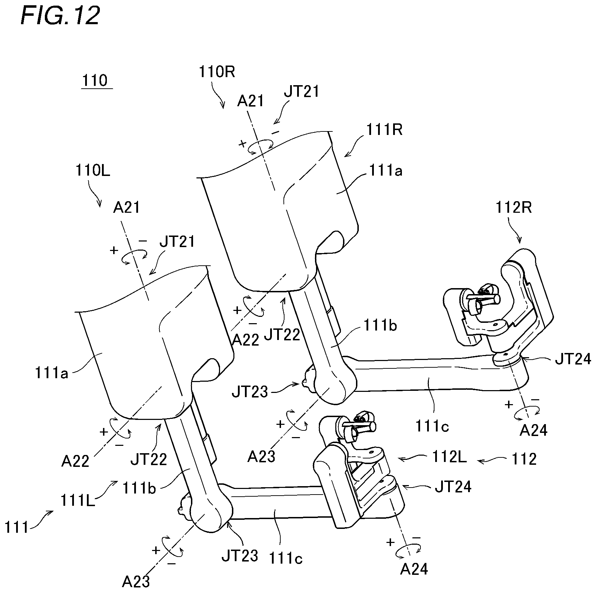

As shown in FIG. 12, the operation units 110 each include a handle to operate the surgical instrument 1. The operation units 110 receive operations of the operator for the surgical instrument 1. The operation units 110 includes an operation unit 110L that is located on the left side as viewed from the operator such as a doctor and is to be operated by the left hand of the operator, and an operation unit 110R that is located on the right side and is to be operated by the right hand of the operator. The operation units 110 each include an arm 111 and a wrist 112. The operation unit 110R includes an arm 111R and a wrist 112R. The operation unit 110L includes an arm 111L and a wrist 112L.

As shown in FIGS. 12, 13, and 14, the operation units 110 have joints JT21, JT22, JT23, JT24, JT25, JT26, and JT27. The rotation axes of the joints JT21, JT22, JT23, JT24, JT25, JT26, and JT27 are A21, A22, A23, A24, A25, A26, and A27 axes, respectively.

### Arm

The arm 111 includes a link 111a, a link 111b, and a link 111c. The upper end side of the link 111a is attached to the remote control apparatus 200 such that the link 111a is rotatable about the A21 axis along a vertical direction. The upper end side of the link 111b is attached to the lower end side of the link 111a such that the link 111b is rotatable about the A22 axis along a horizontal direction. A first end side of the link 111c is attached to the lower end side of the link 111b such that the link 111c is rotatable about the A23 axis along the horizontal direction. The wrist 112 is attached to a second end side of the link 111c such that the wrist 112 is rotatable about the A24 axis. The link 111a is connected to the remote control apparatus 200 by the joint JT21. The link 111a and the link 111b are connected to each other by the joint JT22. The link 111b and the link 111c are connected to each other by the joint JT23. The arm 111 supports the wrist 112.

The wrists 112 includes a wrist 112R shown in FIG. 13 and to be operated by the right hand of the operator, and a wrist 112L shown in FIG. 14 and to be operated by the left hand of the operator. FIG. 13 shows the reference posture of the operation unit 110R, and FIG. 14 shows the reference posture of the operation unit 110L. The configuration of the wrist 112R is the same as or similar to that of the wrist 112L.

The wrist 112 includes a link 112a, a link 112b, a link 112c, and a grip 112d that is gripped and operated by the operator such as a doctor. The link 112a rotates about the A24 axis. The link 112b is attached to the link 112a so as to be rotatable about the A25 axis. The link 112c is attached to the link 112b so as to be rotatable about the A26 axis. The grip 112d is attached to the link 112c so as to be rotatable about the A27 axis. The link 112a, the link 112b, and the link 112c each have an L shape.

The wrist 112 includes a pair of grip members 112e that are opened and closed by the operator. The grip members 112e each include an elongated plate-shaped lever member, and proximal ends of the pair of grip members 112e are rotatably connected to a proximal end of the grip 112d. Cylindrical finger insertion portions 112f are provided on the grip members 112e. The operator inserts their fingers into a pair of finger insertion portions 112f to operate the wrist 112. The proximal ends of the pair of grip members 112e are connected to the grip 112d, and an angle between the pair of grip members 112e is increased or decreased such that an opening angle between the jaw member 2f and the jaw member 2g is changed. A magnet is provided on one of the grip members 112e, and a Hall sensor is provided on the grip 112d. When the operator opens and closes the grip members 112e, the magnet and the Hall sensor function as an angle detection sensor, and the Hall sensor outputs the opening angle. As the angle detection sensor, the Hall sensor may be provided on the grip member 112e, and the magnet may be provided on the grip 112d. Alternatively, the magnet or the Hall sensor may be provided as the angle detection sensor on both the grip members 112e.

The intersection of a plurality of rotation axes of the operation unit 110 is called a gimbal point GP. Specifically, the gimbal point GP is a point at which the A24 axis, the A25 axis, the A26 axis, and the A27 axis intersect with each other. The gimbal point GP is located in the grip 112d to which the pair of grip members 112e are attached. The gimbal point GP exists individually in each of the operation unit 110L and the operation unit 110R. The gimbal point of the operation unit 110R is defined as GPR. The gimbal point of the operation unit 110L is defined as GPL.

In the reference posture, the A24 and A26 axes of the operation unit 110 are along the Zb direction. The A25 axis is along the Xb direction. The A27 axis is along the Yb direction. As shown in FIG. 13, in the reference posture, the link 112a and the link 112b of the wrist 112R are arranged along an Xb-Zb plane and on the Xb1 side with respect to the A27 axis. In the reference posture, the link 112c is arranged along a Yb-Zb plane. In the reference posture, the grip 112d is arranged along the A27 axis.

In the reference posture, the link 112a and the link 112b of the wrist 112L are arranged along the Xb-Zb plane and on the Xb2 side with respect to the A27 axis. In the reference posture, the link 112c is arranged along the Yb-Zb plane. In the reference posture, the grip 112d is arranged along the A27 axis.

As shown in FIG. 1, the monitor 140 is a scope-type display that displays an image captured by the endoscope 3. A notifier 141 is provided on the monitor 140. The notifier 141 issues an error sound. The support arm 150 supports the monitor 140 so as to align the height of the monitor 140 with the height of the face of the operator such as a doctor. The touch panel 130 is arranged on the support bar 160. The head of the operator is detected by a sensor provided in the vicinity of the monitor 140 such that the surgical robot 100 can be operated by the remote control apparatus 200. The operator operates the operation units 110 and the foot pedals 120 while visually recognizing an affected area on the monitor 140. Thus, a command is input to the remote control apparatus 200. The command input to the remote control apparatus 200 is transmitted to the surgical robot 100.

The error reset button 161 is arranged on the support bar 160. The error reset button 161 resets errors in the robotic surgical system 500. The errors may be abnormal deviation errors, for example.

### Foot Pedals

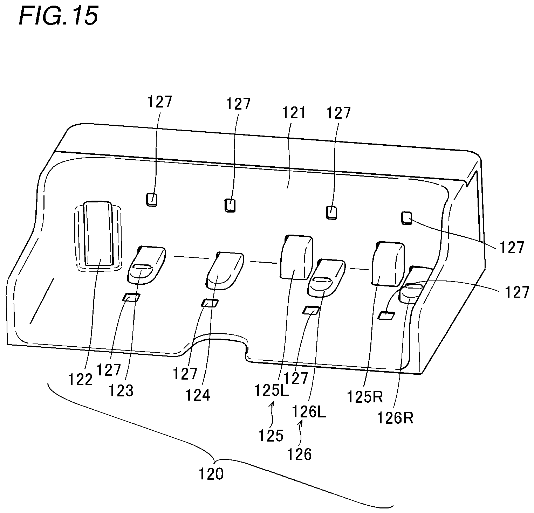

As shown in FIG. 15, a plurality of foot pedals 120 are provided to perform functions related to the surgical instrument 1. The plurality of foot pedals 120 are arranged on a base 121. The foot pedals 120 include a switching pedal 122, a clutch pedal 123, a camera pedal 124, an incision pedal 125, a coagulation pedal 126, and foot detectors 127. The switching pedal 122, the clutch pedal 123, the camera pedal 124, the incision pedal 125, and the coagulation pedal 126 are operated by the foot of the operator. The incision pedal 125 includes an incision pedal 125R for a right robot arm 50, and an incision pedal 125L for a left robot arm 50. The coagulation pedal 126 includes a coagulation pedal 126R for the right robot arm 50 and a coagulation pedal 126L for the left robot arm 50.

The switching pedal 122 switches robot arms 50 to be operated by the operation units 110. The clutch pedal 123 performs a clutch operation to temporarily disconnect an operation connection between the robot arms 50 and the operation units 110. While the clutch pedal 123 is being pressed by the operator, operations by the operation units 110 are not transmitted to the robot arms 50. While the camera pedal 124 is being pressed by the operator, the operation unit 110 can operate a robot arm 50 to which the endoscope 3 is attached. While the incision pedal 125 or the coagulation pedal 126 is being pressed by the operator, an electrosurgical device is activated.

The foot detectors 127 detect the foot of the operator that operates the foot pedals 120. The foot detectors 127 are provided for the switching pedal 122,
the clutch pedal 123, the camera pedal 124, the incision pedal 125L, the coagulation pedal 126L, the incision pedal 125R, and the coagulation pedal 126R, and detect the foot that hovers above their corresponding foot pedals 120. The foot detectors 127 are arranged on the base 121. The functions of the foot pedals 120 including the camera pedal 124 are not limited to being performed through pedals configured to be pressed by the foot of the operator as in this embodiment, but inputs such as hand switches may be provided on the operation units 110, and manual operations by the operator may be used, for example.

### Vision Unit and Image Processing Unit

As shown in FIG. 1, the vision unit 300 and the image processing unit 400 are placed on a cart 210. The image processing unit 400 processes images captured by the endoscope 3. A display 220 is arranged on the cart 210. The display 220 displays images captured by the endoscope 3. An error reset button 230 and a notifier 240 are arranged on the vision unit 300. The error reset button 230 resets errors in the robotic surgical system 500. The errors may be abnormal deviation errors, for example. The notifier 240 issues an error sound.

### Configuration of Control System

As shown in FIG. 16, the robotic surgical system 500 includes a first control device 310, an arm controller 320, a positioner controller 330, operation controllers 340, a second control device 350, and a monitoring controller 360. The robotic surgical system 500 also includes a storage 311 connected to the first control device 310 and the storage 351 connected to the second control device 350.

The first control device 310 is accommodated in the medical cart 10 to communicate with the arm controller 320 and the positioner controller 330, and controls the entire robotic surgical system 500. Specifically, the first control device 310 communicates with and controls the arm controller 320, the positioner controller 330, and the operation controllers 340. The first control device 310 is connected to the arm controller 320, the positioner controller 330, and the operation controllers 340 through a LAN, for example. The first control device 310 is arranged inside the medical cart 10.

The arm controller 320 is arranged for each of the plurality of robot arms 50. That is, the same number of arm controllers 320 as the plurality of robot arms 50 are placed inside the medical cart 10.

The input 22 is connected to the first control device 310 through a LAN, for example. The status indicator 41, the arm status indicator 42, the operation handle 23, the throttle 23a, the joystick 22b, the stabilizer 24, and the electric cylinder 25 are connected to the positioner controller 330 via a wire line 370 by means of a communication network that allows information to be shared with each other by using serial communication. Although FIG. 16 shows that the status indicator 41, the arm status indicator 42, etc. are all connected to one wire line 370, in reality, the wire line 370 is arranged for each of the status indicator 41, the arm status indicator 42, the operation handle 23, the throttle 23a, the joystick 22b, the stabilizer 24, and the electric cylinder 25.

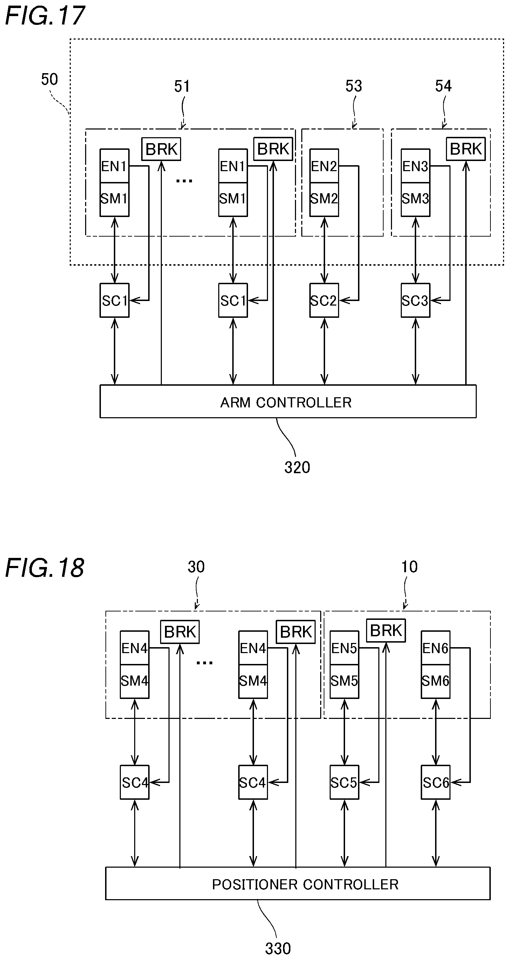

As shown in FIG. 17, the arm portion 51 includes a plurality of servomotors SM1, encoders EN1, and speed reducers so as to correspond to the joints JT1, JT2, JT3, JT4, JT5, JT6, and JT7. The encoders EN1 detect rotation angles of the servomotors SM1. The speed reducers slow down rotation of the servomotors SM1 to increase the torques. Inside the medical cart 10, servo controllers SC1 that control the servomotors SM1 are arranged adjacent to the arm controller 320. In addition, the encoders EN1 that detect the rotation angles of the servomotors SM1 are electrically connected to the servo controllers SC1.

Servomotors SM2 that rotate driven members provided in the driven unit 2a of the surgical instrument 1, encoders EN2, and speed reducers are arranged in the second link 53. The encoders EN2 detect rotation angles of the servomotors SM2. The speed reducers slow down rotation of the servomotors SM2 to increase the torques. In the medical cart 10, servo controllers SC2 are provided to control the servomotors SM2 to drive the surgical instrument 1. The encoders EN2 that detect the rotation angles of the servomotors SM2 are electrically connected to the servo controllers SC2. A plurality of servomotors SM2, a plurality of encoders EN2, and a plurality of servo controllers SC2 are arranged.

The translation mechanism 54 includes a servomotor SM3 to translationally move the surgical instrument 1, an encoder EN3, and a speed reducer. The encoder EN3 detects a rotation angle of the servomotor SM3. The speed reducer slows down rotation of the servomotor SM3 to increase the torque. In the medical cart 10, a servo controller SC3 is provided to control the servomotor SM3 to translationally move the surgical instrument 1. The encoder EN3 that detects the rotation angle of the servomotor SM3 is electrically connected to the servo controller SC3.

The first control device 310 generates command values to command the positions of the servomotors SM1, SM2, and SM3 based on operations received by the remote control apparatus 200, and drives the servomotors SM1, SM2, and SM3 based on the command values. The first control device 310 detects an abnormal deviation error when differences between the command values and the positions of the servomotors SM1, SM2, and SM3 detected by sensors exceed an allowable range.

As shown in FIG. 18, a plurality of servomotors SM4, a plurality of encoders EN4, and a plurality of speed reducers are provided in the positioner 30 so as to correspond to the plurality of joints JT1a, JT2a, JT3a, JT4a, JT5a, JT6a, and JT7a of the positioner 30. The encoders EN4 detect rotation angles of the servomotors SM4. The speed reducers slow down rotation of the servomotors SM4 to increase the torques.

The medical cart 10 includes wheels including front wheels as drive wheels and rear wheels that are steered by the operation handle 23. The rear wheels are arranged closer to the operation handle 23 than the front wheels. The medical cart 10 includes servomotors SM5 to drive a plurality of front wheels of the medical cart 10, encoders EN5, speed reducers, and brakes BRK. The speed reducers slow down rotation of the servomotors SM5 to increase the torques. A potentiometer P1 shown in FIG. 3 is provided on the operation handle 23 of the medical cart 10, and the servomotors SM5 of the front wheels are driven based on a rotation angle detected by the potentiometer P1 according to the twist of the throttle 23a. Rear wheels of the medical cart 10 are of the dual wheel type, and the rear wheels are steered based on rightward-leftward rotation of the operation handle 23. Furthermore, a potentiometer P2 shown in FIG. 3 is provided on a rotation axis of the operation handle 23 of the medical cart 10, and servomotors SM6, encoders EN6, and speed reducers are provided on the rear wheels of the medical cart 10. The speed reducers slow down rotation of the servomotors SM6 to increase the torques. The servomotors SM6 are driven based on a rotation angle detected by the potentiometer P2 according to rightward-leftward rotation of the operation handle 23. That is, steering of the rear wheels by the rightward-leftward rotation of the operation handle 23 is power-assisted by the servomotors SM6.

The front wheels of the medical cart 10 are driven such that the medical cart 10 moves in the forward-rearward direction. Furthermore, the operation handle 23 of the medical cart 10 is rotated such that the rear wheels are steered, and the medical cart 10 turns in the right-left direction.

As shown in FIG. 18, in the medical cart 10, servo controllers SC4 are provided to control the servomotors SM4 to move the positioner 30. The encoders EN4 that detect the rotation angles of the servomotors SM4 are electrically connected to the servo controllers SC4. In the medical cart 10, servo controllers SC5 are provided to control the servomotors SM5 to drive the front wheels of the medical cart 10. The encoders EN5 that detect the rotation angles of the servomotors SM5 are electrically connected to the servo controllers SC5. In the medical cart 10, servo controllers SC6 are provided to control the servomotors SM6 to power-assist steering of the rear wheels of the medical cart 10. The encoders EN6 that detect the rotation angles of the servomotors SM6 are electrically connected to the servo controllers SC6.

As shown in FIGS. 17 and 18, the joints JT1, JT2, JT3, JT4, JT5, JT6, and JT7 of the arm portion 51, and the joints JT1a, JT2a, JT3a, JT4a, JT5a, JT6a, and JT7a of the positioner 30 include brakes BRK. Furthermore, the front wheels of the medical cart 10, the arm base 40, and the translation mechanism 54 include brakes BRK. The arm controller 320 unidirectionally transmits control signals to the brakes BRK of the joints JT1, JT2, JT3, JT4, JT5, JT6, and JT7 of the arm portion 51, and the translation mechanism 54. The control signals are signals for turning on/off the brakes BRK. The signals for turning on the brakes BRK include signals for maintaining the brakes BRK in an enabled state. The same applies to control signals from the positioner controller 330 to the brakes BRK of the joints JT1a, JT2a, JT3a, JT4a, JT5a, JT6a, and JT7a of the positioner 30 and the arm base 40. On startup, all the brakes BRK of the arm base 40, the arm portion 51, and the translation mechanism 54 are turned off but the servomotors SM are driven against gravity to maintain the postures of the robot arm 50 and the arm base 40. When an error occurs in the robotic surgical system 500, the brakes BRK of the arm base 40, the arm portion 51 and the translation mechanism 54 are turned on. When the error in the robotic surgical system 500 is reset, the brakes BRK of the arm base 40, the arm portion 51, and the translation mechanism 54 are turned off. When a shutdown operation is performed in the robotic surgical system 500, the brakes BRK of the arm base 40, the arm portion 51, and the translation mechanism 54 are turned on. The brakes BRK of the front wheels of the medical cart 10 are constantly turned on, and the brakes BRK are deactivated only while the enable switch 23b of the medical cart 10 is being pressed. The brakes BRK of the joints JT1a, JT2a, JT3a, JT4a, JT5a, JT6a, and JT7a of the positioner 30 are constantly turned on, and the brakes BRK are deactivated only while the enable switch 22c of the medical cart 10 is being pressed.

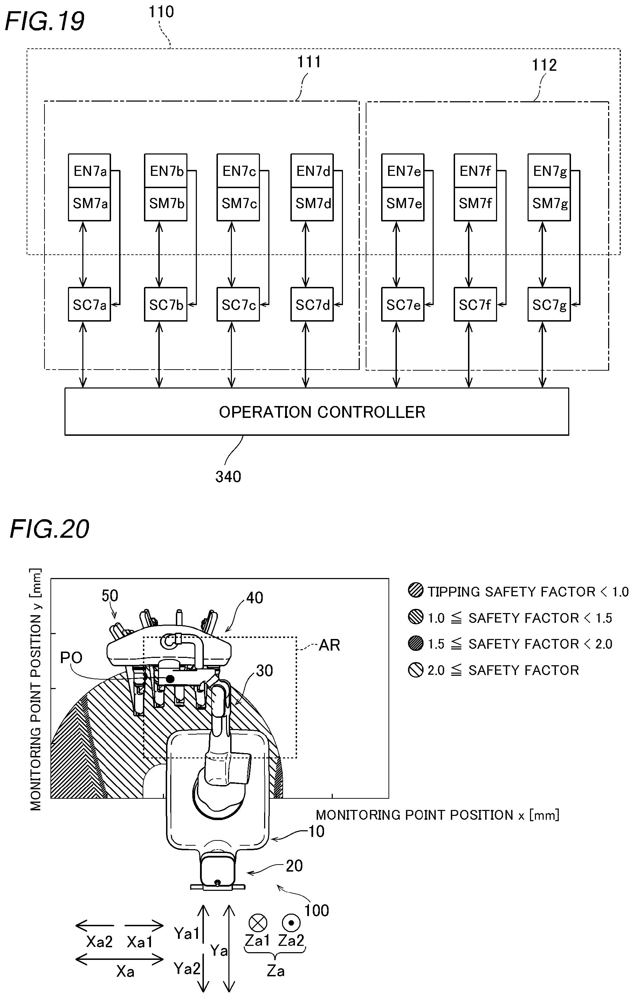

As shown in FIG. 19, servomotors SM7a, SM7b, SM7c, SM7d, SM7e, SM7f, and SM7g are arranged on the joints JT21, JT22, JT23, JT24, JT25, JT26, and JT27 of the operation unit 110, respectively. Servo controllers SC7a, SC7b, SC7c, SC7d, SC7e, SC7f, and SC7g are provided to control the servomotors. Encoders EN7a, EN7b, EN7c, EN7d, EN7e, EN7f, and EN7g are electrically connected to the servo controllers to detect rotation angles of the servomotors. The servomotors, the servo controllers, and the encoders are provided in each of the operation unit 110L and the operation unit 110R. The servomotors SM7a, SM7b, SM7c, SM7d, SM7e, SM7f, and SM7g are provided to assist the operator in performing an operation. The servomotors SM7a, SM7b, SM7c, SM7d, SM7e, SM7f, and SM7g are an example of a drive.

The operation controllers 340 controls the servomotors to generate torques that counteract gravitational torques generated on rotation axes of the servomotors according to the postures of the operation units 110. Thus, the operator can operate the operation units 110 with a relatively small force.

As shown in FIG. 16, the first control device 310 controls the robot arm 50 based on an operation received by the arm operation unit 60. For example, the first control device 310 controls the robot arm 50 based on an operation received by the joystick 62 of the arm operation unit 60. Specifically, the arm controller 320 outputs an input signal input from the joystick 62 to the first control device 310. The first control device 310 generates position commands based on the received input signal and the rotation angles detected by the encoders EN1, and outputs the position commands to the servo controllers SC1 via the arm controller 320. The servo controllers SC1 generate current commands based on the position commands input from the arm controller 320 and the rotation angles detected by the encoders EN1, and output the current commands to the servomotors SM1. Thus, the robot arm 50 is moved according to an operation command input to the joystick 62.

The first control device 310 controls the robot arm 50 based on an input signal from either linear switch 63 of the arm operation unit 60. Specifically, the arm controller 320 outputs the input signal input from the linear switch 63 to the first control device 310. The first control device 310 generates a position command(s) based on the received input signal and the rotation angle(s) detected by the encoders EN1 or the encoder EN3, and outputs the position command(s) to the servo controllers SC1 or the servo controller SC3 via the arm controller 320. The servo controllers SC1 or the servo controller SC3 generates a current command(s) based on the position command(s) input from the arm controller 320 and the rotation angle(s) detected by the encoders EN1 or the encoder EN3, and outputs the current command(s) to the servomotors SM1 or the servomotor SM3. Thus, the robot arm 50 is moved according to an operation command input to the linear switch 63.

The positioner controller 330 is arranged in the medical cart 10. The positioner controller 330 controls the positioner 30 and the medical cart 10. The servomotors SM4, the encoders EN4, and the speed reducers are provided in the positioner 30 so as to correspond to the plurality of joints JT1a, JT2a, JT3a, JT4a, JT5a, JT6a, and JT7a of the positioner 30. The servo controllers SC4 are provided in the medical cart 10 to control the servomotors SM4 of the positioner 30. The servomotors SM5 and SM6 that drive the plurality of front wheels of the medical cart 10, the encoders EN5 and EN6, the speed reducers, the servo controllers SC5 and SC6, and the brakes BRK are provided in the medical cart 10.

The operation controllers 340 are arranged in a main body of the remote control apparatus 200. The operation controllers 340 control the operation units 110. The operation controllers 340 are provided so as to correspond to the left-handed operation unit 110L and the right-handed operation unit 110R, respectively, as shown in FIG. 16. Servomotors SM, encoders EN, and speed reducers are provided in the operation units 110 so as to correspond to a plurality of joints JT21 to JT27 of the operation units 110. Servo controllers SC that control the servomotors SM of the operation units 110 are provided adjacent to the operation controllers 340 in the main body of the remote control apparatus 200.

As shown in FIG. 16, the vision unit 300 and the image processing unit 400 are connected to the first control device 310 through a LAN, for example. The display 220 is connected to the vision unit 300.

The monitoring controller 360 is arranged in the surgical robot 100. The monitoring controller 360 may be arranged in the remote control apparatus 200, or may be arranged independently of the surgical robot 100 and the remote control apparatus 200.

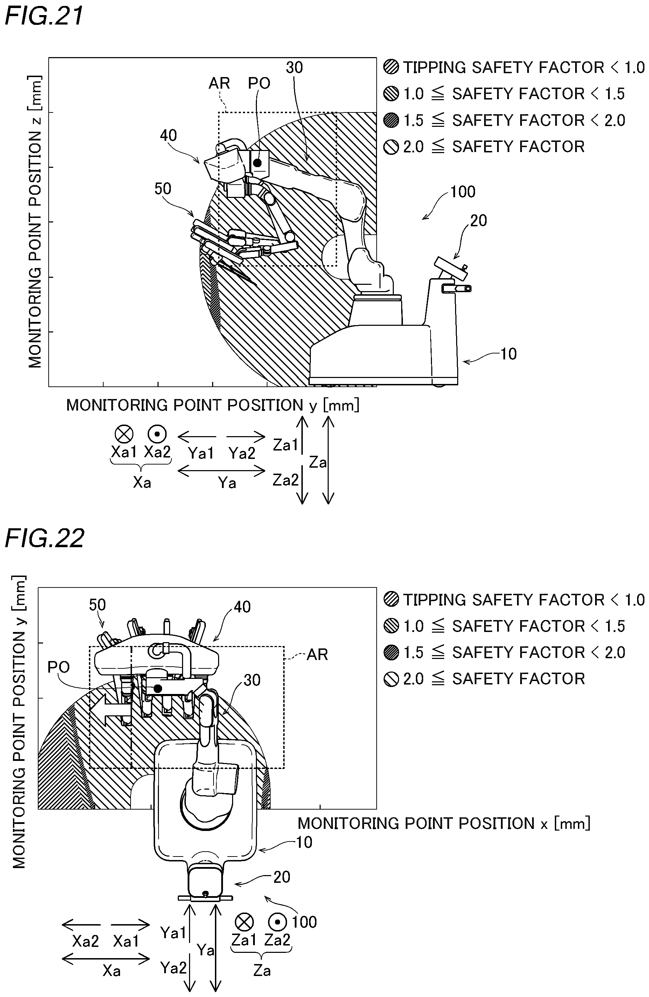

As shown in FIGS. 20 and 21, when the posture of the positioner 30 is set before surgery, for example, the monitoring controller 360 monitors whether or not a monitoring point PO set with respect to the positioner 30 is within a monitoring area AR. When determining that the monitoring point PO is outside the monitoring area AR, the monitoring controller 360 stops the positioner 30 via the positioner controller 330. Thus, the monitoring controller 360 prevents the surgical robot 100 from tipping. The monitoring point PO and the monitoring area AR are set in order to prevent the surgical robot 100 from tipping.

The monitoring point PO is set on the distal end side of the positioner 30. Specifically, the monitoring point PO is set on the A7a axis of the link 32f. The monitoring controller 360 acquires the position of the monitoring point PO based on the rotation angles of the plurality of joints JT1a, JT2a, JT3a, JT4a, JT5a, JT6a, and JT7a of the positioner 30.

The monitoring area AR is set based on a tipping safety factor. The tipping safety factor represents the difficulty of the surgical robot 100 tipping at each position within a movable range of the monitoring point PO. As the tipping safety factor increases, the surgical robot 100 is less likely to tip. The monitoring area AR is set so as not to include an area with a tipping safety factor smaller than a predetermined value, but to include only an area with a tipping safety factor equal to or greater than the predetermined value. The tipping safety factor is a value obtained by dividing the absolute value of the tipping moment of the surgical robot 100 by the absolute value of a tipping moment caused by a reference external force. The tipping moment of the surgical robot 100 is determined by the position of the center of gravity, the weight, and the positions of the wheels of the surgical robot 100. As an example, the monitoring area AR is set to include only an area with a tipping safety factor of 2 or more. In addition, as an example, the reference external force is an external force that applies a horizontal force of 150 N at a height of 1.5 m from the floor.

The monitoring area AR is a three-dimensional area. Specifically, the monitoring area AR is a rectangular parallelepiped area. The monitoring area AR extends in three directions, the Xa direction, the Ya direction, and the Za direction. The center of the monitoring area AR in the Xa direction is located in the vicinity of the center of the medical cart 10 in the Xa direction such that the positioner 30 can take the same or similar posture in both the Xa1 direction and the Xa2 direction. Moreover, the monitoring area AR is located on the Ya1 direction side and the Za1 direction side with respect to the base 31.

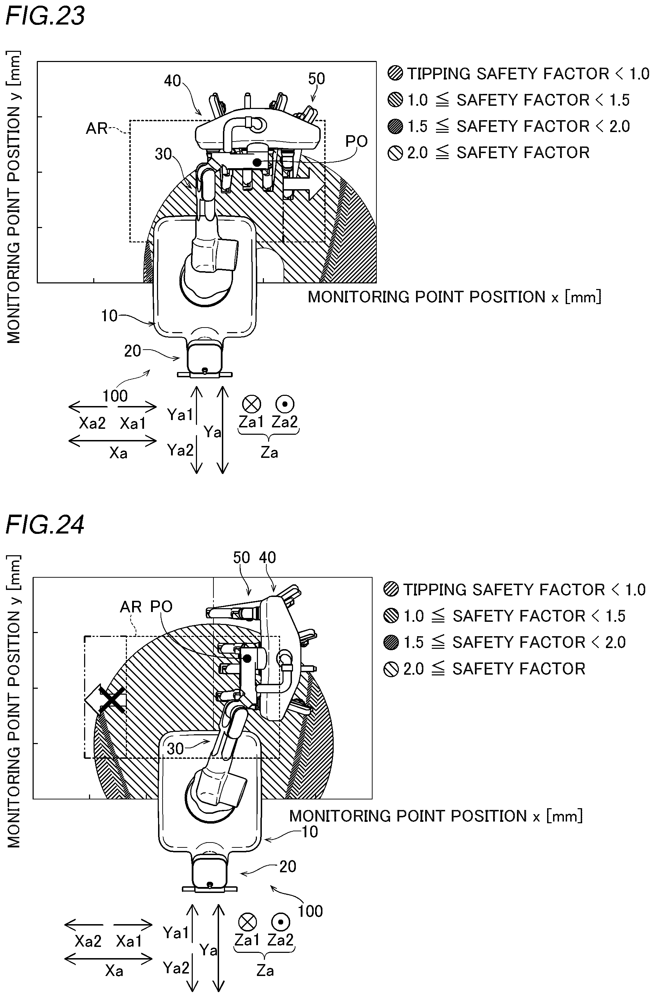

In this embodiment, as shown in FIGS. 22 and 23, the monitoring controller 360 changes the monitoring area AR based on the posture of the positioner 30. Specifically, the monitoring controller 360 expands the monitoring area AR based on the posture of the positioner 30. The monitoring controller 360 expands the monitoring area AR in the Xa direction. In addition, the monitoring controller 360 expands the monitoring area AR so as not to include an area with a tipping safety factor smaller than a predetermined value, but to include only an area with a tipping safety factor equal to or greater than the predetermined value.

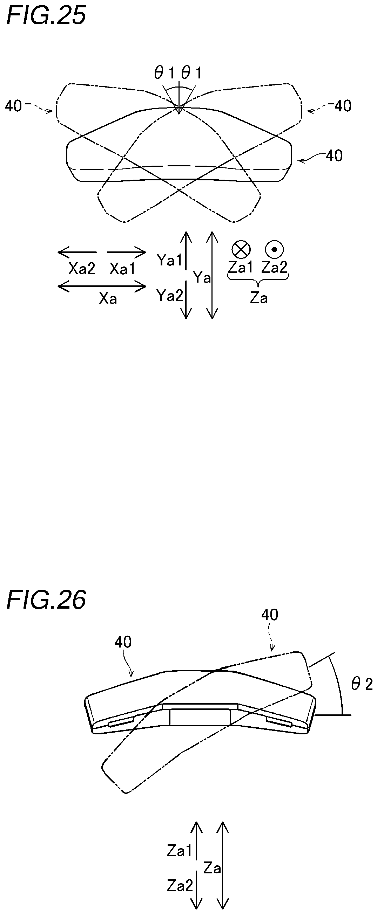

In this embodiment, the monitoring controller 360 expands the monitoring area AR based on the rotation angle Θ1 of the arm base 40 around a vertical axis, which is based on the posture of the positioner 30. The rotation angle Θ1 of the arm base 40 around the vertical axis is a rotation angle of the arm base 40 about an axis in the Za direction with respect to the arm base 40 in the reference posture, as shown in FIG. 25. The monitoring controller 360 acquires the rotation angle Θ1 of the arm base 40 around the vertical axis based on the rotation angles of the plurality of joints JT1a, JT2a, JT3a, JT4a, JT5a, JT6a, and JT7a of the positioner 30.

In this embodiment, as shown in FIG. 24, the monitoring controller 360 does not expand the monitoring area AR when the rotation angle Θ1 of the arm base 40 around the vertical axis is greater than a first threshold. As shown in FIGS. 22 and 23, the monitoring controller 360 expands the monitoring area AR when the rotation angle Θ1 of the arm base 40 around the vertical axis is equal to or smaller than the first threshold. The first threshold is not particularly limited, but is 45 degrees, for example.

In this embodiment, the monitoring controller 360 expands the monitoring area AR in a direction corresponding to rotation of the joint JT7a on the distal end side connected to the arm base 40 among the plurality of joints JT1a, JT2a, JT3a, JT4a, JT5a, JT6a, and JT7a. Specifically, when the joint JT7a on the distal end side is rotated to a first side, the monitoring controller 360 expands the monitoring area AR to a side corresponding to the first side, and when the joint JT7a on the distal end side is rotated to a second side, the monitoring controller 360 expands the monitoring area AR to a side corresponding to the second side. More specifically, when the rotation angle of the joint JT7a on the distal end side is smaller than 0, the monitoring controller 360 determines that the joint JT7a on the distal end side has been rotated to the first side, and expands the monitoring area AR to the Xa2 direction side. When the rotation angle of the joint JT7a on the distal end side is equal to or greater than 0, the monitoring controller 360 determines that the joint JT7a on the distal end side has been rotated to the second side, and expands the monitoring area AR to the Xa1 direction side.

As shown in FIG. 22, when the rotation angle Θ1 of the arm base 40 around the vertical axis is equal to or smaller than the first threshold and the rotation angle of the joint JT7a on the distal end side is smaller than 0, the positioner 30 takes a posture extending in the Xa2 direction. Therefore, the monitoring controller 360 expands the monitoring area AR to the Xa2 direction side within a range that does not include an area with a tipping safety factor smaller than the predetermined value but includes only an area with a tipping safety factor equal to or greater than the predetermined value. As shown in FIG. 23, when the rotation angle Θ1 of the arm base 40 around the vertical axis is equal to or smaller than the first threshold and the rotation angle of the joint JT7a on the distal end side is equal to or greater than 0, the positioner 30 takes a posture extending in the Xa1 direction. Therefore, the monitoring controller 360 expands the monitoring area AR to the Xa1 direction side within a range that does not include an area with a tipping safety factor smaller than the predetermined value but includes only an area with a tipping safety factor equal to or greater than the predetermined value. In these cases, the monitoring controller 360 monitors whether or not the monitoring point PO is within the expanded monitoring area AR.

On the other hand, as shown in FIG. 24, when the rotation angle Θ1 of the arm base 40 around the vertical axis is greater than the first threshold, the positioner 30 takes a posture extending in the Ya1 direction. In an example shown in FIG. 24, the rotation angle of the joint JT7a on the distal end side is smaller than 0, but when the monitoring area AR is expanded to the Xa2 direction side based on the rotation angle of the joint JT7a on the distal end side, the expanded monitoring area AR includes an area with a tipping safety factor smaller than the predetermined value. Therefore, when the rotation angle Θ1 of the arm base 40 around the vertical axis is greater than the first threshold, the monitoring controller 360 does not expand the monitoring area AR.

In this embodiment, the monitoring controller 360 determines that the monitoring point PO is outside the monitoring area AR when the inclination angle Θ2 of the arm base 40 with respect to horizontal based on the posture of the positioner 30 is equal to or greater than a second threshold. The inclination angle Θ2 of the arm base 40 with respect to horizontal is a rotation angle of the arm base 40 about a horizontal axis with respect to the arm base 40 in the reference posture, as shown in FIG. 26. The monitoring controller 360 acquires the inclination angle Θ2 of the arm base 40 with respect to horizontal based on the rotation angles of the plurality of joints JT1a, JT2a, JT3a, JT4a, JT5a, JT6a, and JT7a of the positioner 30. The second threshold is not particularly limited, but is 90 degrees, for example. When the inclination angle Θ2 of the arm base 40 with respect to horizontal is smaller than the second threshold, the monitoring controller 360 monitors whether or not the monitoring point PO is within the monitoring area AR as usual.

### Advantages of This Embodiment

According to this embodiment, as described above, the monitoring controller 360 is provided to monitor whether or not the monitoring point PO set with respect to the arm base 40 or the positioner 30 is within the monitoring area AR. Accordingly, the monitoring controller 360 monitors whether or not the monitoring point PO set with respect to the arm base 40 or the positioner 30 is within the monitoring area AR, and thus the positioner 30 can take a posture within a range in which the surgical robot 100 can maintain its balance. Consequently, the surgical robot 100 can be prevented from tipping due to the posture of the positioner 30. Furthermore, the monitoring area AR is changed based on the posture of the positioner 30, and thus the positioner 30 can take more postures as compared with a case in which the monitoring area AR is not changed. Consequently, the positioner 30 can take more postures while the surgical robot 100 is prevented from tipping due to the posture of the positioner 30.

According to this embodiment, as described above, the monitoring controller 360 is configured or programmed to expand the monitoring area AR based on the posture of the positioner 30. Accordingly, the positioner 30 can take a posture such that the monitoring point PO is within the expanded monitoring area AR. Consequently, the monitoring area AR is expanded such that the positioner 30 can take more postures. Furthermore, a wider operating range can be ensured.

According to this embodiment, as described above, the monitoring controller 360 is configured or programmed to expand the monitoring area AR based on the rotation angle Θ1 of the arm base 40 around the vertical axis, which is based on the posture of the positioner 30. Accordingly, when it is better to expand the monitoring area AR based on the rotation angle Θ1 of the arm base 40 around the vertical axis, the monitoring area AR can be expanded. When it is better not to expand the monitoring area AR based on the rotation angle Θ1 of the arm base 40 around the vertical axis, the monitoring area AR can be prevented from being expanded. Consequently, the monitoring area AR can be appropriately expanded based on the rotation angle Θ1 of the arm base 40 around the vertical axis.

According to this embodiment, as described above, the monitoring controller 360 is configured or programmed to not expand the monitoring area AR when the rotation angle Θ1 of the arm base 40 around the vertical axis is greater than the first threshold, and to expand the monitoring area AR when the rotation angle Θ1 of the arm base 40 around the vertical axis is equal to or smaller than the first threshold. Accordingly, the monitoring area AR can be easily expanded or shifted by using the rotation angle Θ1 of the arm base 40 around the vertical axis and the first threshold.

According to this embodiment, as described above, the positioner 30 includes the plurality of joints JT1a, JT2a, JT3a, JT4a, JT5a, JT6a, and JT7a, and the monitoring controller 360 is configured or programmed to expand the monitoring area AR in the direction corresponding to rotation of the joint JT7a on the distal end side connected to the arm base 40 among the plurality of joints JT1a, JT2a, JT3a, JT4a, JT5a, JT6a, and JT7a. Accordingly, the monitoring area AR can be expanded or shifted in an appropriate direction by using the fact that the rotation of the joint JT7a on the distal end side corresponds to the direction in which the monitoring area AR is expanded.

According to this embodiment, as described above, the monitoring controller 360 is configured or programmed to expand the monitoring area AR to the side corresponding to the first side when the joint JT7a on the distal end side is rotated to the first side, and expand the monitoring area AR to the side corresponding to the second side when the joint JT7a on the distal end side is rotated to the second side. Accordingly, the monitoring area AR can be easily expanded in an appropriate direction by using the fact that the rotation direction of the joint JT7a on the distal end side corresponds to the direction in which the monitoring area AR is expanded.

According to this embodiment, as described above, the monitoring controller 360 is configured or programmed to determine that the monitoring point PO is outside the monitoring area AR when the inclination angle θ2 of the arm base 40 with respect to horizontal, which is based on the posture of the positioner 30, is equal to or greater than the second threshold. Accordingly, in an abnormal case in which the arm base 40 is rotated excessively as compared with a normal state in which the robot arm 50 is present on the lower side, such as when the arm base 40 is inverted, it can be determined that the monitoring point PO is outside the monitoring area AR.

According to this embodiment, as described above, the robotic surgical system 500 includes the remote control apparatus 200 for the operator to operate the robot arm 50. Accordingly, the remote control apparatus 200 allows the operator to easily operate the robot arm 50.

According to this embodiment, as described above, the robotic surgical system 500 includes the cart positioner operation unit 20 for the operator to operate the positioner 30. Accordingly, the cart positioner operation unit 20 allows the operator to easily operate the positioner 30. Furthermore, when the positioner 30 is operated using the cart positioner operation unit 20 to change the posture of the positioner 30, it is possible to monitor whether or not the monitoring point PO is within the monitoring area AR, and thus it is possible to prevent the surgical robot 100 from tipping due to the posture of the positioner 30.

According to this embodiment, as described above, the monitoring area AR is a three-dimensional area. Accordingly, the positioner 30 can take more postures as compared with a case in which the monitoring area AR is a planar area. When the positioner 30 can take more postures, it is particularly effective to prevent the surgical robot 100 from tipping due to the posture of the positioner 30.

### Modified Examples

The embodiments disclosed this time must be considered as illustrative in all points and not restrictive. The scope of the present disclosure is not shown by the above description of the embodiments but by the scope of claims for patent, and all modifications or modified examples within the meaning and scope equivalent to the scope of claims for patent are further included.

For example, while the monitoring point PO is set with respect to the positioner 30 in the aforementioned embodiment, the present disclosure is not limited to this. For example, the monitoring point PO may alternatively be set with respect to the arm base 40.

While the monitoring area AR is expanded based on the posture of the positioner 30 in the aforementioned embodiment, the present disclosure is not limited to this. For example, the monitoring area AR may alternatively be shifted based on the posture of the positioner 30. In such a case, when the joint JT7a on the distal end side is rotated to the first side, the monitoring area AR may be shifted to the side corresponding to the first side, and when the joint JT7a on the distal end side is rotated to the second side, the monitoring area AR may be shifted to the side corresponding to the second side.

While the monitoring area AR is expanded in the Xa direction in the aforementioned embodiment, the present disclosure is not limited to this. For example, the monitoring area AR may alternatively be expanded or shifted in a direction other than the Xa direction, such as the Ya direction or the Za direction.

While the monitoring area AR is a rectangular parallelepiped area in the aforementioned embodiment, the present disclosure is not limited to this. For example, the monitoring area AR may be an area other than a rectangular parallelepiped area, such as a spherical area.

While the cart positioner operation unit 20 is provided as a second operation apparatus according to the present disclosure in the aforementioned embodiment, the present disclosure is not limited to this. As the second operation apparatus according to the present disclosure, a dedicated operation apparatus may alternatively be provided for the operator to operate the positioner 30.

While the monitoring area AR is expanded based on the rotation angle θ1 of the arm base 40 around the vertical axis in the aforementioned embodiment, the present disclosure is not limited to this. For example, the monitoring area AR may not be expanded based on the rotation angle θ1 of the arm base 40 around the vertical axis.

While the monitoring area AR is expanded in the direction corresponding to rotation of the joint JT7a on the distal end side in the aforementioned embodiment, the present disclosure is not limited to this. For example, the monitoring area AR may not be expanded in the direction corresponding to rotation of the joint JT7a on the distal end side.

While four robot arms 50 are provided in the aforementioned embodiment, the present disclosure is not limited to this. In the present disclosure, the number of robot arms 50 may be any number as long as at least one robot arm 50 is provided.

While each of the arm portion 51 and the positioner 30 includes a 7-axis articulated robot in the aforementioned embodiment, the present disclosure is not limited to this. For example, each of the arm portion 51 and the positioner 30 may alternatively include an articulated robot having an axis configuration other than the 7-axis articulated robot. The axis configuration other than the 7-axis articulated robot includes six axes or eight axes, for example.

The functionality of the elements disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out the recited functionality or hardware that is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor.

### Aspects

It will be appreciated by those skilled in the art that the exemplary embodiments described above are specific examples of the following aspects.

### (Item 1)

A robotic surgical system comprising:
a surgical apparatus including a first robot arm to move a medical instrument, an arm base to support a proximal end of the first robot arm, and a second robot arm to move the arm base; and
a monitoring controller configured or programmed to monitor whether or not a monitoring point set with respect to the arm base or the second robot arm is within a monitoring area, and change the monitoring area based on a posture of the second robot arm.

### (Item 2)

The robotic surgical system according to item 1, wherein the monitoring controller is configured or programmed to expand or shift the monitoring area based on the posture of the second robot arm.

### (Item 3)

The robotic surgical system according to item 2, wherein the monitoring controller is configured or programmed to expand or shift the monitoring area based on a rotation angle of the arm base around a vertical axis, which is based on the posture of the second robot arm.

### (Item 4)

The robotic surgical system according to item 3, wherein the monitoring controller is configured or programmed to not expand or shift the monitoring area when the rotation angle of the arm base around the vertical axis is greater than a first threshold, and to expand or shift the monitoring area when the rotation angle of the arm base around the vertical axis is equal to or smaller than the first threshold.

### (Item 5)

The robotic surgical system according to item 2 or 3, wherein
the second robot arm includes a plurality of joints; and
the monitoring controller is configured or programmed to expand or shift the monitoring area in a direction corresponding to rotation of a joint on a distal end side connected to the arm base among the plurality of joints.

### (Item 6)

The robotic surgical system according to item 5, wherein the monitoring controller is configured or programmed to expand or shift the monitoring area to a side corresponding to a first side when the joint on the distal end side is rotated to the first side, and expand or shift the monitoring area to a side corresponding to a second side when the joint on the distal end side is rotated to the second side.

### (Item 7)

The robotic surgical system according to any one of items 1 to 6, wherein the monitoring controller is configured or programmed to determine that the monitoring point is outside the monitoring area when an inclination angle of the arm base with respect to horizontal, which is based on the posture of the second robot arm, is equal to or greater than a second threshold.

### (Item 8)

The robotic surgical system according to any one of items 1 to 7, further comprising:
a first operation apparatus for an operator to operate the first robot arm.

### (Item 9)

The robotic surgical system according to any one of items 1 to 8, further comprising:
a second operation apparatus for an operator to operate the second robot arm.

### (Item 10)

The robotic surgical system according to any one of items 1 to 9, wherein the monitoring area is a three-dimensional area.

### (Item 11)

A surgical apparatus comprising:
a first robot arm to move a medical instrument;
an arm base to support a proximal end of the first robot arm;
a second robot arm to move the arm base; and a monitoring controller configured or programmed to monitor whether or not a monitoring point set with respect to the arm base or the second robot arm is within a monitoring area, and change the monitoring area based on a posture of the second robot arm.

### (Item 12)

A control method for a robotic surgical system, the robotic surgical system comprising a surgical apparatus including a first robot arm to move a medical instrument, an arm base to support a proximal end of the first robot arm, and a second robot arm to move the arm base, the control method comprising:
monitoring whether or not a monitoring point set with respect to the arm base or the second robot arm is within a monitoring area; and
changing the monitoring area based on a posture of the second robot arm.

## Claims

1. A robotic surgical system (500) comprising:
a surgical apparatus (100) including a first robot arm (50) to move a medical instrument (1), an arm base (40) to support a proximal end of the first robot arm (50), and a second robot arm (30) to move the arm base (40); and
a monitoring controller (360) configured or programmed to monitor whether or not a monitoring point (PO) set with respect to the arm base (40) or the second robot arm (30) is within a monitoring area (AR), and change the monitoring area (AR) based on a posture of the second robot arm (30).

2. The robotic surgical system (500) according to claim 1, wherein the monitoring controller (360) is configured or programmed to expand or shift the monitoring area (AR) based on the posture of the second robot arm (30) .

3. The robotic surgical system (500) according to claim 2, wherein the monitoring controller (360) is configured or programmed to expand or shift the monitoring area (AR) based on a rotation angle (θ1) of the arm base (40) around a vertical axis, which is based on the posture of the second robot arm (30).

4. The robotic surgical system (500) according to claim 3, wherein the monitoring controller (360) is configured or programmed to not expand or shift the monitoring area (AR) when the rotation angle (θ1) of the arm base (40) around the vertical axis is greater than a first threshold, and to expand or shift the monitoring area (AR) when the rotation angle (θ1) of the arm base (40) around the vertical axis is equal to or smaller than the first threshold.

5. The robotic surgical system (500) according to claim 2 or 3, wherein
the second robot arm (30) includes a plurality of joints (JT1a, JT2a, JT3a, JT4a, JT5a, JT6a, JT7a); and
the monitoring controller (360) is configured or programmed to expand or shift the monitoring area (AR) in a direction corresponding to rotation of a joint (JT7a) on a distal end side connected to the arm base (40) among the plurality of joints (JT1a, JT2a, JT3a, JT4a, JT5a, JT6a, JT7a) .

6. The robotic surgical system (500) according to claim 5, wherein the monitoring controller (360) is configured or programmed to expand or shift the monitoring area (AR) to a side corresponding to a first side when the joint (JT7a) on the distal end side is rotated to the first side, and expand or shift the monitoring area (AR) to a side corresponding to a second side when the joint (JT7a) on the distal end side is rotated to the second side.

7. The robotic surgical system (500) according to any one of claims 1 to 6, wherein the monitoring controller (360) is configured or programmed to determine that the monitoring point (PO) is outside the monitoring area (AR) when an inclination angle (θ2) of the arm base (40) with respect to horizontal, which is based on the posture of the second robot arm (30), is equal to or greater than a second threshold.

8. The robotic surgical system (500) according to any one of claims 1 to 7, further comprising:
a first operation apparatus (200) for an operator to operate the first robot arm (50).

9. The robotic surgical system (500) according to any one of claims 1 to 8, further comprising:
a second operation apparatus (20) for an operator to operate the second robot arm (30).

10. The robotic surgical system (500) according to any one of claims 1 to 9, wherein the monitoring area (AR) is a three-dimensional area.

11. The robotic surgical system (500) according to any one of claims 1 to 10, wherein the monitoring area (AR) includes only an area with a tipping safety factor equal to or greater than a predetermined value.

12. The robotic surgical system (500) according to claim 11, wherein the monitoring area (AR) has a rectangular parallelepiped shape.

13. A control method for a robotic surgical system (500), the robotic surgical system (500) comprising a surgical apparatus (100) including a first robot arm (50) to move a medical instrument (1), an arm base (40) to support a proximal end of the first robot arm (50), and a second robot arm (30) to move the arm base (40), the control method comprising:
monitoring whether or not a monitoring point (PO) set with respect to the arm base (40) or the second robot arm (30) is within a monitoring area (AR); and
changing the monitoring area (AR) based on a posture of the second robot arm (30).
